# EUROPEAN PATENT APPLICATION

(11) **EP 4 520 378 A1**
(43) Date of publication of application: **12.03.2025**
(21) Application number: 23195881.0
(22) Date of filing: 07.09.2023
(51) Int. Cl.: A61M 16/16, A61M 16/00, A61M 16/08

(54) **RESPIRATORY SUPPORT DEVICE, METHOD, COMPUTER PROGRAM PRODUCT, AND WATER COLLECTION UNIT**

(71) Applicant: Koninklijke Philips N.V., 5656 AG Eindhoven (NL)
(72) Inventor: VAN DEN ENDE, Daan Anton, Eindhoven (NL)
(74) Representative: Philips Intellectual Property & Standards

(57) **Abstract**

There is provided a respiratory support device for providing air to a subject via a patient interface. The respiratory support device comprises a conduit system, a humidifier, a blower, and a water collection unit. The conduit system has a first outlet configured to be coupled to the patient interface. The humidifier is adapted to humidify a first flow of air. The blower is arranged to generate a second flow of air. The water collection unit is adapted to collect water from the second flow of air. The conduit system is adapted to convey the first flow of air via the humidifier to the first outlet. The conduit system is adapted to convey the second flow of air via the water collection unit. Further, there is provided a method for using a respiratory support device.

## Description

### FIELD OF THE INVENTION

The invention relates to a respiratory support device for providing air to a subject via a patient interface. The invention further relates to a method for using a respiratory support device for providing air to a subject via a patient interface. The invention further relates to a computer program product comprising instructions to be executed by a control unit of the respiratory support device. The invention further relates to a humidifier for use in the respiratory support device. The invention further relates to a water collection unit for use in the respiratory support device.

### BACKGROUND OF THE INVENTION

Sleep-disordered breathing (SDB) conditions, such as OSA and CSA, are becoming increasingly common, and are particularly prevalent in older people, people with a high body mass index, smokers, heavy drinkers and people with conditions such as coronary artery disease, hypertension and diabetes mellitus.

SDB conditions are often treated using positive airway pressure (PAP) therapy, in which a pressurized flow of air is provided to a subject to keep the subject's airways open. When first prescribing PAP therapy, a PAP titration study is carried out for the subject in order to determine a level of airway pressure to be provided to the subject during PAP therapy, as well as a suitable PAP therapy modality (e.g. continuous positive airway pressure, CPAP, bilevel positive airway pressure, BiPAP, or automatic positive airway pressure, APAP) and a suitable patient interface (e.g. a nasal pillow, an oronasal/full-face mask).

The pressurized flow of air is provided by a respiratory support device. A patient interface is connected to the respiratory support device to convey the flow of air to the subject. The flow of air is at least partly inhaled by the subject.

To improve the comfort for the subject, it is known to humidify the flow of air before providing the flow of air to the subject. By humidifying the flow of air, the risk of irritating the throat or nose of the subject is reduced. To humidify the flow of air, some types of respiratory support devices are provided with a humidifier. The humidifier has a water reservoir that needs to have a minimum amount of water for the humidifier to be able to humidify the flow of air.

### SUMMARY OF THE INVENTION

For known respiratory support devices, the subject needs to ensure that the water reservoir remains sufficiently filled with water. In many cases, this means refilling the water reservoir with tap water every one or two days. The problem arises that people forget to refill the water reservoir. This has the result that the subject receives uncomfortably dry air from the respiratory support device, resulting in irritations. Using a respiratory support device is not easy for many people. The irritations caused by the dry air provide an additional difficulty for people to adhere to the therapy. In a worst case, the irritations cause people to stop using the respiratory support device.

The subject may not notice that the water reservoir runs out of water, in case this happens during the night, while the subject is sleeping. Many people that use a respiratory support device, have difficulties sleeping. Those people may be exhausted when preparing to go to bed, and may be more likely to forget checking or refilling the water reservoir.

It is an objective of the invention to improve the comfort of using a respiratory support device.

According to a first aspect of the invention, there is provided a respiratory support device for providing air to a subject via a patient interface, the respiratory support device comprising:
a conduit system having a first outlet configured to be coupled to the patient interface;
a humidifier adapted to humidify a first flow of air;
a blower arranged to generate a second flow of air;
a water collection unit adapted to collect water from the second flow of air,
wherein the conduit system is adapted to convey the first flow of air via the humidifier to the first outlet; and
wherein the conduit system is adapted to convey the second flow of air via the water collection unit.

According to a second aspect of the invention, there is provided a method for using a respiratory support device for providing air to a subject via a patient interface, the method comprising:
generating a first flow of air via a first outlet coupled to the patient interface;
using a blower to generate a second flow of air;
collecting water from the second flow of air;
humidifying the first flow of air with the water.

According to a third aspect of the invention, there is provided a computer program product, comprising instructions which, when executed by a control unit of a respiratory support device according to the first aspect, cause the respiratory support device to perform the method of the second aspect.

According to a fourth aspect of the invention, there is provided a humidifier for use in the respiratory support device according to the first aspect.

According to a fifth aspect of the invention, there is provided a water collection unit for use in the respiratory support device according to the first aspect.

### BRIEF DESCRIPTION OF THE DRAWINGS

Exemplary embodiments will now be described, by way of example only, with reference to the following figures, in which:
FIG. 1 depicts a first embodiment according to the invention;
FIG. 2A depicts a detail of the first embodiment in a first mode;
FIG. 2B depicts the detail of the first embodiment in a second mode;
FIG. 3 depicts a second embodiment according to the invention;
FIG. 4 depicts a third embodiment according to the invention;
FIG. 5 depicts a fourth embodiment according to the invention;
FIG. 6 depicts a humidifier for use in any of the mentioned embodiments;
FIG. 7 depicts a water collection unit for use in any of the mentioned embodiments;
FIG. 8 depicts a control unit for use in any of the mentioned embodiments;
FIG. 9 depicts a method for using a respiratory support device according to the second aspect of the invention.

### DETAILED DESCRIPTION OF EMBODIMENTS

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

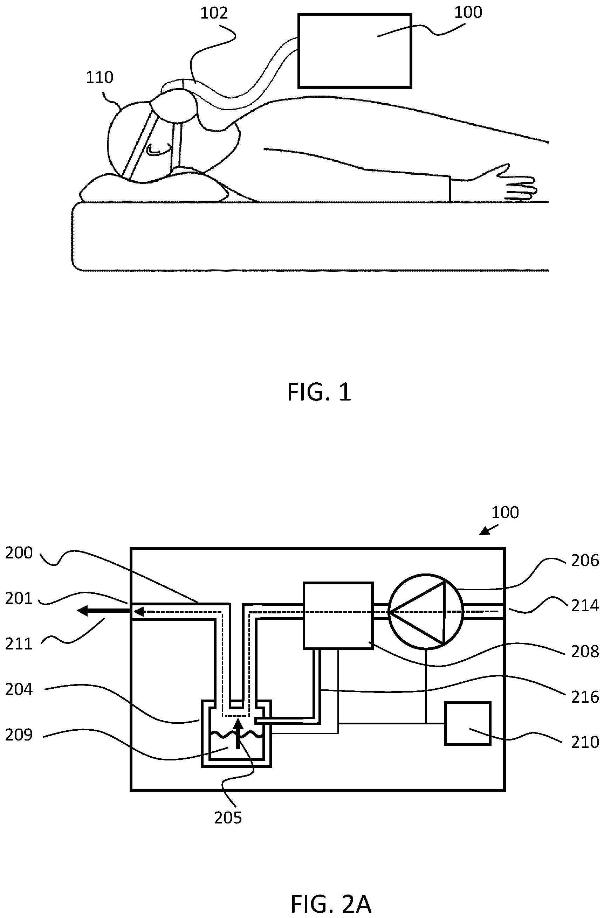

FIG. 1 depicts a first embodiment according to the invention. In the first embodiment, a respiratory support device 100 is provided. The respiratory support device 100 is for providing air to a subject 110 via a patient interface 102. The patient interface 102 comprises a tube and a face mask. Straps are provided to secure the face mask to the face of the subject 110. While the subject 110 is sleeping, the respiratory support device 100 helps to reduce or prevent SDB events, such as apnea events or hypopnea events. The respiratory support device 100 helps to reduce the Apnea-Hypopnea Index, AHI. The subject 110 is able to breath in the air provided by the respiratory support device 100 via the tube and the face mask.

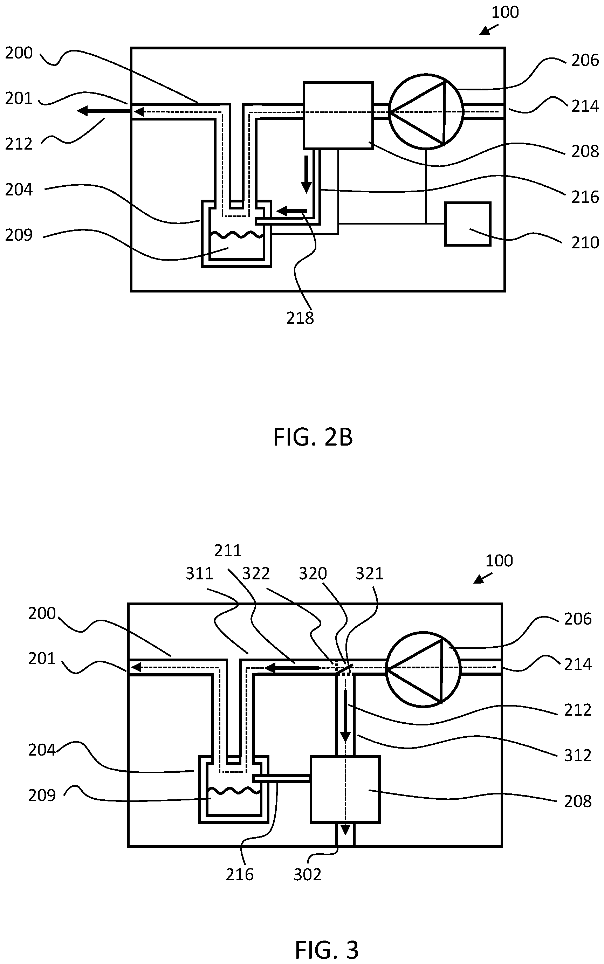

FIGs. 2A and 2B depict the respiratory support device 100 according to the first embodiment. The respiratory support device 100 is for providing air to a subject 110 via the patient interface 102. The respiratory support device 100 comprises a conduit system 200, a humidifier 204, a blower 206 and a water collection unit 208. The conduit system 200 has a first outlet 201 configured to be coupled to the patient interface 102. The humidifier 204 is adapted to humidify a first flow of air 211. The blower 206 is arranged to generate a second flow of air 212. The water collection unit 208 is adapted to collect water from the second flow of air 212. The conduit system 200 is adapted to convey the first flow of air 211 via the humidifier 204 to the first outlet 201. The conduit system 200 is adapted to convey the second flow of air 212 via the water collection unit 208.

The respiratory support device 100 provides the first flow of air 211 to the subject 110 via the first outlet 201. The first flow of air 211 flows via the humidifier 204. The humidifier 204 humidifies the first flow of air 211 towards the subject 110 to provide humidified air to the subject 110. Humified air improves the comfort for the subject 110 receiving the humidified flow of air. The humidifier 204 requires water to humidify the first flow of air 211. Water is collected via the water collection unit 208 from the second flow of air 212. The second flow of air 212 is not provided to the subject 110. This allows the water collection unit 208 to remove water vapor from the air without the risk of providing dry air to the subject 110. Therefore, the water collection unit 208 is able to collect water to be used by the humidifier 204 without negatively affecting the subject 110. Because the water for the humidifier 204 is, at least partly, provided by the water collection unit 208, the operation of the respiratory support device 100 is less dependent or not dependent at all on water from an external supply. This improves to comfort of using the respiratory support device 100.

For example, the respiratory support device 100 is a PAP device, such as a CPAP, BiPAP or auto-PAP. For example, the patient interface 102 comprises a face mask, or a nasal mask.

The conduit system 200 is, for example, a single conduit for conveying the first flow of air 211 and the second flow of air 212. In another example, the conduit system 200 comprises multiple conduits that are interconnected. The conduit system 200 comprises, in an example, multiple conduits that are separated from each other and are not interconnected to each other. The conduit system 200 comprises, for example, tubes and/or hoses. For example, at least a portion of the conduit system 200 is formed by a housing of the respiratory support device 100, such as by a hollow section of the respiratory support device 100.

The blower 206 is arranged to generate the second flow of air 212. For example, the blower 206 has a fan or an impeller to displace air to generate the second flow of air 212. For example, the blower 206 comprises an electromotor to drive the fan or to drive the impeller. The blower 206 is, for example, a centrifugal air pump, or an axial air pump. The blower 206 is, for example, a reciprocating air pump or a rotary displacement air pump.

The water collection unit 208 is adapted to collect water from the second flow of air 212. The second flow of air 212 contains water vapor. The second flow of air 212 originates, for example, from an ambient environment. Under some conditions, such as in a dry environment or during a dry, sunny day, the second flow may contain only little water vapor. Under other conditions, such as in a humid environment or during a rainy day, the second flow may contain a lot of water vapor. Depending on the amount of water vapor in the second flow of air 212, the water collection unit 208 collects water from a large second flow of air 212 or a small second flow of air 212 to collect a desired amount of water.

For example, the water collection unit 208 comprises a condenser to condense water vapor from the second flow of air 212. A condenser provides a surface to be in contact with the second flow of air 212. The surface causes the water vapor in the second flow of air 212 to condensate into water droplets. The water droplets are collected for use in the humidifier 204. For example, the surface has a temperature that is lower than the temperature of the second flow of air 212. For example, the surface is passively cooled by providing a heat sink to transfer heat away from the surface. For example, the surface is actively cooled by transferring heat from the surface to a heat transfer fluid that is circulated by a pump. For example, the heat transfer fluid is water or a refrigerant or a coolant.

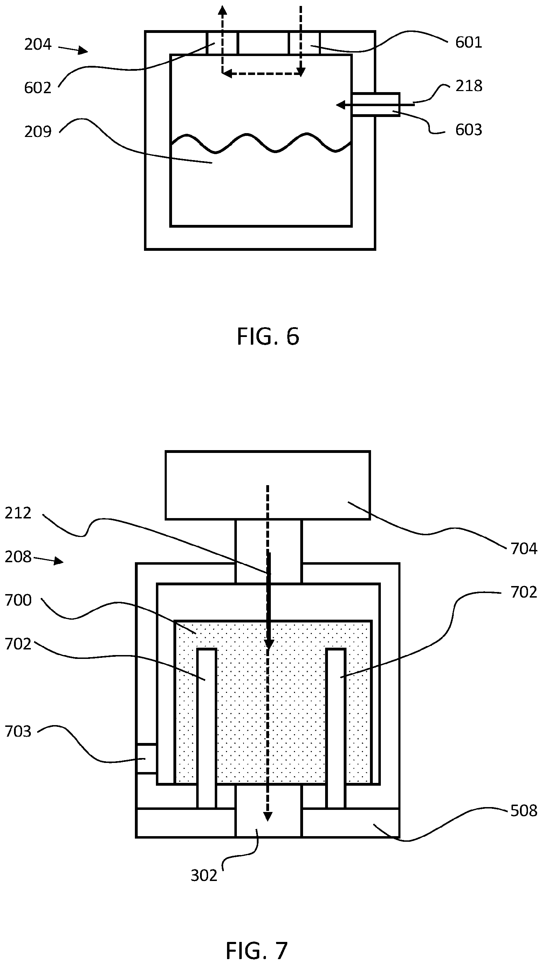

For example, as shown in FIG. 7, the water collection unit 208 comprises hygroscopic material 700 adapted to adsorb water vapor from the second flow of air 212. The respiratory support device 100 comprises a heater 506 arranged, via heat conductor 508, to heat the hygroscopic material 700 to either remove adsorbed water vapor from the hygroscopic material 700 or to adsorb water in the hygroscopic material 700. In an alternative embodiment, no heater 506 is arranged to heat the hygroscopic material 700, but instead a cooler is provided to cool the hygroscopic material 700. In an alternative embodiment, the heater 506 is replaced by a thermoelectric device, such as a Peltier element, adapted to both heat and cool. For example, the thermoelectric device is arranged to heat and cool the hygroscopic material 700. For example, the thermoelectric device provides heat to the hygroscopic material 700 to release water from the hygroscopic material 700, and extracts heat (i.e., cools) from the hygroscopic material 700 to adsorb water vapor from the second flow of air 212, or vice versa.

Hygroscopic material 700 has the property that it adsorbs water from a flow of air in a certain temperature range, and that it releases the adsorbed water at a different temperature range. So by repeatedly heating and cooling the hygroscopic material 700, water vapor is adsorbed by the hygroscopic material 700, and a desired amount of water is collected from the hygroscopic material 700.

For example, the hygroscopic material 700 comprises at least one of a hygroscopic hydrogel, a hygroscopic salt, paper coated with lithium chloride, paper coated with or calcium, and wool.

As shown in FIGs. 2A and 2B, the respiratory support device 100 comprises a water conduit 216 connecting the water collection unit 208 and the humidifier 204 to each other. The water conduit 216 is arranged to provide water from the water collection unit 208 to the humidifier 204. Alternatively, the respiratory support device 100 does not comprise the water conduit 216. In this alternative, the water collection unit 208 comprises, for example, a receptacle to collect the water. The subject 110 needs to manually use the receptacle to fill the water reservoir 209 of the humidifier 204. This increases the comfort for the subject 110 compared to the known art. The subject 110 may be able to refill the water reservoir 209 while in bed. There is no need to go to the bathroom or the kitchen to get water for the humidifier 204. This is especially beneficial when the subject 110 wakes up during the night and notices that the water reservoir 209 is empty.

Further, FIGs. 2A and 2B depict that the respiratory support device 100 comprises a control unit 210. FIG. 2A depicts a first mode of the control unit 210. FIG. 2B depicts a second mode of the control unit 210. The blower 206 is arranged to generate the first flow of air 211 and the second flow of air 212. The conduit system 200 is adapted to convey the second flow of air 212 to the first outlet 201. The control unit 210 is configured to operate the respiratory support device 100 in a first mode and a second mode. The first mode and the second mode are temporally separated. In the first mode, as shown in FIG. 2A, the control unit 210 controls the blower 206 to generate the first flow of air 211, and controls the humidifier 204 to humidify the first flow of air 211. In the second mode, as shown in FIG. 2B, the control unit 210 controls the blower 206 to generate the second flow of air 212, and controls the water collection unit 208 to collect water from the second flow of air 212.

In the first mode, the subject 110 is able to use the respiratory support device 100. A vapor flow 205 from the humidifier 204 is mixed with the first flow of air 211 to humidify the first flow of air 211. For example, the water in the water reservoir 209 is heated to increase the vapor flow 205. Preferably, the control unit 210 disables the water collection unit 208 to prevent the water collection unit 208 to collect water from the first flow of air 211.

The respiratory support device 100 is not used by the subject 110 the whole day, but for example only while sleeping at night. When the respiratory support device 100 is not used by the subject 110, the control unit 210 operates the respiratory support device 100 in the second mode. In the second mode, the blower 206 generates the second flow of air 212, which is conveyed along the same flow path as the first flow of air 211. The flow path is indicated with the dashed arrow. In the second mode, the control unit 210 operates the water collection unit 208 to collect water from the second flow of air 212. For example, the control unit 210 activates a condenser in the water collection unit 208. For example, the control unit 210 controls repeated heating and cooling of hygroscopic material 700 in the water collection unit 208. The control unit 210 disables the humidifier 204, for example by not heating the water in the water reservoir 209, or for example, operating a valve to have the second flow of air 212 bypass at least part of the humidifier 204. For example, the control unit 210 disables the humidifier 204 by controlling the blower 206 to generate the second flow of air 212 at a higher flow rate than the first flow of air 211. Because of the higher flow rate, the second flow of air 212 does not spent sufficient time in the humidifier 204 to become significantly humified by the humidifier 204.

The water collection unit 208 collects water, and a flow of water 218 is provided from the water collection unit 208 via the water conduit 216 to the humidifier 204.

FIG. 3 depicts a second embodiment according to the invention. The second embodiment is, for example, the same as the first embodiment, except for the following.

The respiratory support device 100 according to the second embodiment has the conduit system 200. The conduit system 200 comprises a first conduit 311 and a second conduit 312. The first conduit 311 is adapted to convey the first flow of air 211 via the humidifier 204 to the first outlet 201. The second conduit 312 system 200 is adapted to convey the second flow of air 212 via the water collection unit 208.

The second conduit 312 has a second outlet 302 to an ambient environment. The respiratory support device 100 comprises a valve 320 coupling the first conduit 311 and the second conduit 312 to each other. The valve 320 is configured to be in a first position 321 and in a second position 322.

In the first position 321, the valve 320 allows the first flow of air 211 through the first conduit 311 to the first outlet 201, and blocks the second flow of air 212 from flowing through the second conduit 312. In the second position 322, the valve 320 allows the second flow of air 212 through the second conduit 312 and the water collection unit 208, and blocks the first flow of air 211 from flowing through the first outlet 201.

When the subject 110 is using the respiratory support device 100, the valve 320 is in the first position 321. When the valve 320 is in the first position 321, the first flow of air 211 is able to flow from the blower 206 via the valve 320 to the humidifier 204 and to the first outlet 201. No flow is possible from the blower 206 via the valve 320 through the second conduit 312 and through the water collection unit 208. When the subject 110 is not using the respiratory support device 100, the valve 320 is in the second position 322. When the valve 320 is in the second position 322, the second flow of air 212 is able to flow from the blower 206 via the valve 320 to the water collection unit 208 and to the second outlet 302. No flow is possible from the blower 206 via the valve 320 through the first conduit 311 and through the humidifier 204 to the first outlet 201. Because the second outlet 302 is to the ambient environment, it is prevented that the subject 110 receives the dry second flow of air 212 via the patient interface 102. Further, in case one or more air filters are provided downstream of the blower 206, it is advantageous to place some or all of the air filters downstream of the valve 320 in the first conduit 311. This way, the first flow of air 211 is sufficiently filtered. Because the second flow of air 212 is directed to the second outlet 302 to the ambient environment, less filtering or no filtering is needed. This way, the amount of flow through the air filters is limited to only the first flow of air 211. This increases the time between replacing or cleaning the air filters.

The conduit system 200 comprises an inlet 214. The conduit system 200 is adapted to convey the first flow of air 211 from the inlet 214 via the valve 320 to the humidifier 204, and from the humidifier 204 to the first outlet 201. The conduit system 200 is adapted to convey the second flow of air 212 from the inlet 214 via the valve 320 to the water collection unit 208.

Alternatively to the second embodiment, no valve 320 is provided. In this alternative embodiment, the blower 206 generates the first flow of air 211 and the second flow of air 212 simultaneously. This allows the water collection unit 208 to collect water from the second flow of air 212, while the first flow of air 211 is humidified and is provided to the subject 110. Although this leads to a simpler design without the valve 320, the flow capacity of the blower 206 needs to be larger than in the second embodiment, because the blower 206 needs to generate both the first flow of air 211 and the second flow of air 212 simultaneously. In the second embodiment, the blower 206 only needs to generate the first flow of air 211 and the second flow of air 212 one at a time.

In a further alternative embodiment to the second embodiment, no second outlet 302 is provided. Instead, the second flow of air 212 exiting from the water collection unit 208 is conveyed by the conduit system 200 to the first outlet 201. The respiratory support device 100 only provides the second flow of air 212 when the subject 110 is not using the respiratory support device 100. This allows the dry second flow of air 212 to exit via the first outlet 201 without negatively affecting the subject 110.

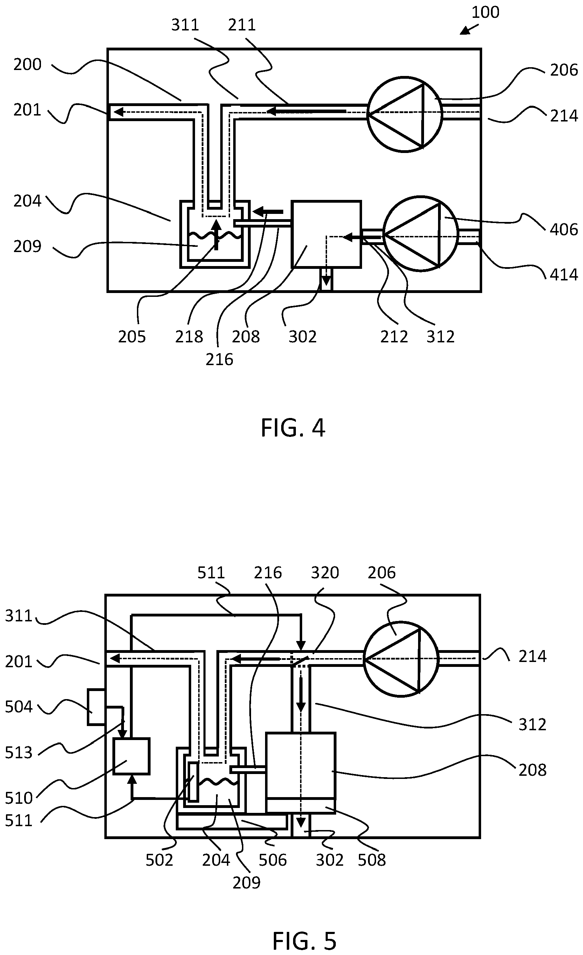

FIG. 4 depicts a third embodiment according to the invention. The third embodiment is, for example, the same as the first embodiment or the same as the second embodiment, except for the following.

The respiratory support device 100 according to the third embodiment has the first conduit 311 and the second conduit 312 that are separated from each other. The respiratory support device 100 comprises the blower 206 to generate the first flow of air 211 from the first inlet 214 via the humidifier 204 to the first outlet 201. The respiratory support device 100 comprises a second blower 406 to generate the second flow of air 212 from a second inlet 414 via the water collection unit 208 to the second outlet 302.

This embodiment has the advantage that each of the flow path of the first flow of air 211, and the flow path of the second flow of air 212 can be optimized. The flow paths are indicated by dashed arrows. For example, the blower 206 is adapted to generate the first flow of air 211 at various flow rates corresponding to desired therapy flow rates. For example, the blower 206 is designed to operate very silently, because the subject 110 is sleeping while the blower 206 is in use. The first conduit 311 is, for example, provided with one or more filters, to ensure an excellent air quality of the first flow of air 211. For example, the second blower 406 is adapted to generate the second flow of air 212 at only one flow rate. For example, the second blower 406 is a relatively cheap blower designed to generate a high flow rate. For example, the flow rate of the second blower 406 is matched with an optimal flow rate for the water collection unit 208. For example, the water collection unit 208 collects water in the most efficient way when the second flow of air 212 is at the optimal flow rate. Because the second blower 406 is used when the subject 110 is not using the respiratory support device 100, it may be acceptable that the second blower 406 makes noise.

FIG. 5 depicts a fourth embodiment according to the invention. The fourth embodiment is, for example, the same as the second embodiment, except for the following.

The respiratory support device 100 comprises a water control unit 510 and a water level sensor 502. The water level sensor 502 is configured to generate a water level signal 511 representative of a water level in the humidifier 204. The water control unit 510 is configured to receive the water level signal 511. The water control unit 510 is configured to provide a valve control signal 512 to the valve 320 in response to the water level signal 511 to control a position of the valve 320.

When the water control unit 510 receives the water level signal 511 from the water level sensor 502, and determines based on the water level signal 511 that more water is desired in the water reservoir 209, the water control unit 510 provides the valve control signal 512 to the valve 320 to position the valve 320 in the second position 322. The water control unit 510 controls the blower 206 to generate the second flow of air 212 from the first inlet 214 via the valve 320 to the water collection unit 208. The water control unit 510 controls the water collection unit 208 to collect water from the second flow of air 212. The flow of water 218 via the water conduit 216 is received in the water reservoir 209.

When the water control unit 510 receives the water level signal 511 from the water level sensor 502, and determines based on the water level signal 511 that no more water is desired in the water reservoir 209, the water control unit 510 controls the blower 206 to stop generating the second flow of air 212. Optionally, the water control unit 510 controls the water collection unit 208 to stop collecting water from the second flow of air 212. Optionally, the water control unit 510 provides the valve control signal 512 to position the valve 320 in the first position 321.

For example, the water level sensor 502 is a conductive level sensor or a capacitive level sensor or an optical level sensor. For example, the water level sensor 502 comprises a float switch. For example, the water level sensor 502 is adapted to generate a binary water level signal 511. The binary water level signal 511 indicates either that sufficient water is present in the water reservoir 209 or that insufficient water is present in the water reservoir 209. In another example, water level sensor 502 is adapted to generate a water level signal 511 for a plurality of water levels. For example, the water level sensor 502 is adapted to generate a water level signal 511 proportional to the water level, between a minimum level and a maximum level.

In an example, the water control unit 510 is configured to provide the valve control signal 512 to position the valve 320 in the first position 321 in case the water level signal 511 represents a water level above a threshold level. The water control unit 510 is configured to provide the valve control signal 512 to position the valve 320 in the second position 322 in case the water level signal 511 represents a water level below a threshold level. For example, the threshold level is a single value. When the water level is below the threshold level, the water control unit 510 controls the blower 206 and the valve 320 to collect water till the water level in the water reservoir 209 reaches the threshold level. After the water level reaches the threshold level, the water control unit 510 stops controlling the blower 206 and the valve 320 to collect water. In another example, the threshold level has a maximum level and a minimum level. When the water level is below the minimum level, the water control unit 510 controls the blower 206 and the valve 320 to collect water till the water level in the water reservoir 209 reaches the maximum level. After the water level reaches the maximum level, the water control unit 510 stops controlling the blower 206 and the valve 320 to collect water. For example, the maximum level is more than 50% or more than 75% or about 100% of the water capacity of the water reservoir 209. For example, the minimum level is less than 50% or less than 25% of the water capacity of the water reservoir 209. It is preferred to have sufficient water at the minimum level, because it may be not preferred to collect water while the subject 110 is using the respiratory support device 100. Preferably, the minimum level is sufficient to operate the humidifier 204 until the subject 110 stops using the respiratory support device 100.

The fourth embodiment shows the following optional features. The respiratory support device 100 comprises a user interface 504. The water control unit 510 is configured to receive a use signal 513 representative of the subject 110 is about to use or uses the respiratory support device 100. In response to receiving the use signal 513, the water control unit 510 provides the valve control signal 512 to position the valve 320 in the first position 321.

Via the user interface 504, the subject 110 indicates that he or she is about to use the respiratory support device 100. For example, the user interface 504 is a switch or a button. For example, the user interface 504 comprises a touch screen. For example, the user interface 504 is activated remotely via a remote control or a mobile device, for example via Wi-Fi or Bluetooth. In another example, the user interface 504 is integrated in the patient interface 102. When the subject 110 places the patient interface 102 to his or her face, the user interface 504 is automatically activated. For example, the user interface 504 comprises a sensor in the patient interface 102, such as a pressure sensor arranged to detect a pressure between the patient interface 102 and the face of the subject 110, a heat sensor arranged to detect body heat of the subject 110, or any other type of sensor adapted to detect whether the subject 110 has placed the patient interface 102 on his or her face. When the water control unit 510 receives the use signal 513 from the user interface 504, the water control unit 510 determines the subject 110 is about to use the respiratory support device 100. In response, the water control unit 510 sends the valve control signal 512 to the valve 320 to position the valve 320 in the first position 321.

FIG. 5 further depicts that the humidifier 204 comprises the water reservoir 209 for storing water. A heater 506 is arranged to provide heat to the water reservoir 209 to generate water vapor. For example, the heater 506 is an electric heater.

In this embodiment, the water collection unit 208 comprises hygroscopic material 700 configured to adsorb water vapor from the second flow of air 212. As an optional feature, the heater 506 is arranged to also heat the hygroscopic material 700 to either remove adsorbed water vapor from the hygroscopic material 700 or to adsorb water in the hygroscopic material 700. As shown in FIG. 5, the heater 506 extends beyond the humidifier 204. The water collection unit 208 has a heat conductor 508 that is arranged to come into physical contact with the heater 506. For example, the heat conductor 508 comprises aluminium or copper or any other metal with a high thermal conductivity. For example, the heat conductor 508 comprises aluminum nitride or silicon carbide or silicon nitride or any other ceramic with a high thermal conductivity. In an alternative embodiment, the heater 506 does not extend beyond the humidifier 204, but instead the heat conductor 508 extends to the heater 506.

The embodiments described above make use of the same inventive concept. The first flow of air 211 is humidified and is provided to the subject 110. At least part of the water needed for humifying the first flow of air 211 is obtained from the second flow of air 212. As a result of removing water from the second flow of air 212, the second flow of air 212 becomes dryer. To prevent the subject 110 to be negatively affected by uncomfortably dry air, the first flow of air 211 and the second flow of air 212 are separated from each other. The first flow of air 211 and the second flow of air 212 are separated from each other in time, i.e., temporally, as is done in the first embodiment, for example. In the first embodiment, at a certain time, the first flow of air 211 is provided, whereas at another time, the second flow of air 212 is provided. The first flow of air 211 and the second flow of air 212 are separated from each other in location, as is done, for example, in the third embodiment. In the third embodiment, the first flow of air 211 flows through the first outlet 201, whereas the second flow of air 212 flows through the second outlet 302. The first flow of air 211 and the second flow of air 212 are separated from each other both in time and in location, as is done, for example, in the second embodiment. In the second embodiment, at a certain time, the first flow of air 211 is provided via the first conduit 311, whereas at another time, the second flow of air 212 is provided via the second conduit 312.

FIG. 6 depicts a humidifier 204 for use in any of the mentioned embodiments. The humidifier 204 comprises a humidifier inlet 601 and a humidifier outlet 602. The humidifier inlet 601 is adapted to be coupled to the conduit system 200, for example, to the first conduit 311, to allow the first flow of air 211 to enter the humidifier 204. The humidifier outlet 602 is adapted to be coupled to the conduit system 200, for example, to the first conduit 311, to allow the first flow of air 211 to exit the humidifier 204. The flow path of the first flow of air 211 is indicted with the dashed arrows. The humidifier 204 comprises a water inlet 603 to receive the flow of water 218 from the water collection unit 208 via the water conduit 216.

The humidifier 204 may be any type of humidifier 204 that is suitable for use in the respiratory support device 100. The humidifier 204 is schematically depicted as a pass-over type humidifier 204. The first flow of air 211 passes over a free surface of water in the humidifier 204. The free surface of water generates vapor. The vapor is absorbed by the first flow of air 211. For example, the humidifier 204 is another pass-over type humidifier 204, such as a wick type humidifier or a membrane type humidifier. For example, the type of the humidifier 204 is a bubble through humidifier (BTH). In a BTH, the humidifier inlet 601 is under water in the water reservoir 209. The BTH receives the first flow of air 211 in the water in the water reservoir 209 via a diffuser. The diffuser divides the first flow of air 211 into small bubbles. The bubbles travel through the water in the water reservoir 209 upward, while the bubbles are humidified by the water. When the bubbles are above the water level, they leave the humidifier 204 via the humidifier outlet 602 as the first flow of air 211.

FIG. 7 depicts an example of a water collection unit 208 for use in any of the mentioned embodiments. The water collection unit 208 comprises hygroscopic material 700, a heat conductor 508, heat conductor protrusions 702, a water outlet 703, the second outlet 302, and a coupling device 704.

The hygroscopic material 700 is configured to adsorb water vapor from the second flow of air 212. At room temperature, the hygroscopic material 700 is able to adsorb water vapor from the second flow of air 212.

By heating the hygroscopic material 700, the adsorbed water is removed from the hygroscopic material 700. The water then flows, for example due to gravity or caused by a pump, through the water outlet 703 to the water conduit 216. Alternatively, at room temperature the adsorbed water is removed from the hygroscopic material 700. By heating the hygroscopic material 700, the hygroscopic material 700 is able to adsorb water vapor from the second flow of air 212. This depends on the type of hygroscopic material 700 that is used.

The type of hygroscopic material 700 is, for example, a thermo-responsive hygrogel. A thermo-responsive hygrogel alternates between a hydrophilic and hydrophobic state around a transition temperature. The thermos-response is positive, causing swelling at a temperature above the transition temperature, or negative, causing swelling below the transition temperature. For example, a well-known hygroscopic material is, poly(N-isopropylacrylamide), abbreviated as PNIPAAm. PNIPAAm is water adsorbing at a temperature below the transition temperature, and expels water at a temperature above the transition temperature. For example, the transition temperature is 32°C, but the transition temperature is chemically tuneable. In another type of hygroscopic material is a Poly Ethylene Glycol based hygrogel, which is water adsorbing at a temperature above the transition temperature, and expels water at a temperature below the transition temperature.

For example, the hygroscopic material 700 comprises at least one of a hygroscopic hydrogel, a hygroscopic salt, paper coated with lithium chloride, paper coated with or calcium, and wool.

The heat conductor 508 is arranged to be in thermal contact with the heater 506. To divide the heat along a larger part of the hygroscopic material 700, heat conductor protrusions 702 are provided on the heat conductor 508. The heat conductor protrusions 702 transfer heat from the heat conductor 508 into the hygroscopic material 700 and/or vice versa. For example, the heat conductor protrusions 702 are pins or fins or plates. In addition or alternative to using the heat conductor protrusions 702, the heater 506 is arranged to generate a flow of hot air through the hygroscopic material 700. For example, the heater 506 is arranged to heat the second flow of air 212.

In an embodiment, the water collection unit 208 comprises a sponge adapted to adsorb water vapor from the second flow of air 212 flowing through or along the sponge. By compressing the sponge, the water adsorbed in the sponge is released. For example, the water collection unit 208 comprises a compressing device adapted to compress the sponge. For example, the subject 110 is able to manually operate the compressing device to compress the sponge. Alternatively, the compressing device comprises an actuator adapted to compress the sponge. The actuator is controlled by the water control unit 510.

The coupling device 704 is adapted to couple with the conduit system 200. The water collection unit 208 is adapted to allow the second flow of air 212 to flow through the coupling device 704 via the hygroscopic material 700 to the second outlet 302 arranged in the water collection unit 208. The hygroscopic material 700 is, for example, a porous material that allows the second flow of air 212 to pass through. For example, through holes are provided in the hygroscopic material 700 for the second flow of air 212 to pass through. For example, the water collection unit 208 comprises the second conduit 312. For example, the coupling device 704 is adapted to couple with the first conduit 311.

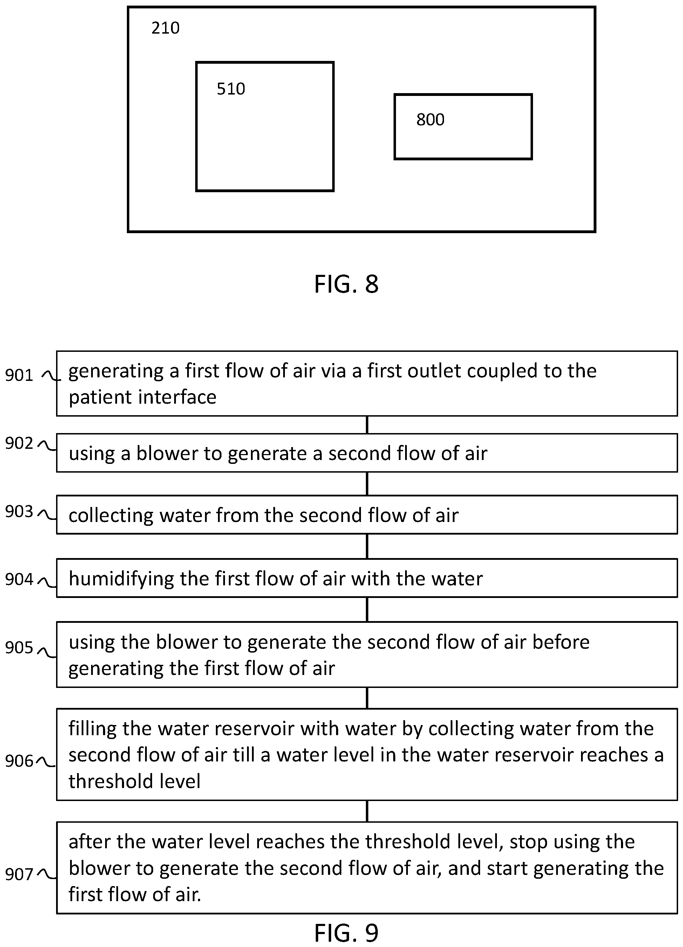

FIG. 8 depicts a control unit 210 for use in any of the mentioned embodiments. As described in the embodiments above, the control unit 210 is configured to operate the respiratory support device 100 in the first mode and the second mode. Optionally, the control unit 210 comprises the water control unit 510. The water control unit 510 is configured to provide the valve control signal 512 to the valve 320 in response to the water level signal 511 to control a position of the valve 320. Optionally, the water control unit 510 is configured to receive the use signal 513 and, in response to receiving the use signal 513, provide the valve control signal 512 to position the valve 320 in the first position 321. The water control unit 510 is, for example, implemented as a separate module within the control unit 210. In another example, the water control 510 is integrated with the control unit 210. For example, the control 210 is a single control unit configured to provide the functions of both the control unit 210 and the water control unit 510.

The control unit 210 comprises a storage medium 800, such as a memory. The storage medium 800 is configured to store a computer program product. The computer program product comprises instructions which, when executed by the control unit 210 of the respiratory support device 100 operates the respiratory support device 100 according to the embodiments mentioned above. For example, the computer program product comprises instructions which, when executed by the control unit 210 of the respiratory support device 100, cause the respiratory support device 100 to perform the method according to the second aspect of the invention as described below.

FIG. 9 depicts a method for using a respiratory support device 100 according to the second aspect of the invention. The method is for using a respiratory support device 100 for providing air to a subject 110 via a patient interface 102. The method comprises steps 901-904. Step 901 comprise generating a first flow of air 211 via a first outlet 201 coupled to the patient interface 102. Step 902 comprises using a blower 206 to generate a second flow of air 212. Step 903 comprises collecting water from the second flow of air 212. Step 904 comprises humidifying the first flow of air 211 with the water.

Optionally, the method comprises steps 905-907. Step 905 comprises using the blower 206 to generate the second flow of air 212 before generating the first flow of air 211. Step 906 comprises filling the water reservoir 209 with water by collecting water from the second flow of air 212 till a water level in the water reservoir 209 reaches a threshold level. Step 907 comprises, after the water level reaches the threshold level, stop using the blower 206 to generate the second flow of air 212, and start generating the first flow of air 211.

In the third aspect of the invention, there is provided a computer program product. The computer program comprises instructions which, when executed by the control unit 210 of a respiratory support device 100 according to any one of the embodiments mentioned above, cause the respiratory support device 100 to perform the method of FIG.9.

It will be understood that the method may be a computer-implemented method. As such, there is also proposed a concept of a computer program comprising code means for implementing the described method when said program is run on a processing system. For example, the control unit 210 comprises a processing system for executing at least some of the instructions of the computer program product. For example, the water control unit 510 comprises a processing system for executing at least some of the instructions of the computer program product. The skilled person would be readily capable of developing such a processing system. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in such a processing system include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Functions implemented by a processing system may be implemented by a single processor or by multiple separate processors units which may together be considered to constitute a "processor". Such processing units may in some cases be remote from each other and communicate with each other in a wired or wireless manner.

The storage medium 800 may be any suitable medium to store the computer program, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain features are recited in mutually different dependent claims does not indicate that a combination of these features cannot be used to advantage. Any reference signs in the claims should not be construed as limiting the scope.

## Claims

1. A respiratory support device (100) for providing air to a subject (110) via a patient interface (102), the respiratory support device (100) comprising:
a conduit system (200) having a first outlet (201) configured to be coupled to the patient interface (102);
a humidifier (204) adapted to humidify a first flow of air (211);
a blower (206) arranged to generate a second flow of air (212);
a water collection unit (208) adapted to collect water from the second flow of air (212),
wherein the conduit system (200) is adapted to convey the first flow of air (211) via the humidifier (204) to the first outlet (201); and
wherein the conduit system (200) is adapted to convey the second flow of air (212) via the water collection unit (208).

2. The respiratory support device (100) according to claim 1, comprising a control unit (210),
wherein the blower (206) is arranged to generate the first flow of air (211) and the second flow of air (212),
wherein the conduit system (200) is adapted to convey the second flow of air (212) to the first outlet (201),
wherein control unit (210) is configured to operate the respiratory support device (100) in a first mode and a second mode,
wherein the first mode and the second mode are temporally separated,
wherein, in the first mode, the control unit (210) controls the blower (206) to generate the first flow of air (211), and controls the humidifier (204) to humidify the first flow of air (211),
wherein, in the second mode, the control unit (210) controls the blower (206) to generate the second flow of air (212), and controls the water collection unit (208) to collect water from the second flow of air (212).

3. The respiratory support device (100) according to claim 1, wherein the conduit system (200) comprises a first conduit (311) and a second conduit (312),
wherein the first conduit (311) is adapted to convey the first flow of air (211) via the humidifier (204) to the first outlet (201); and
wherein the second conduit (312) system (200) is adapted to convey the second flow of air (212) via the water collection unit (208).

4. The respiratory support device (100) of according to claim 3, wherein the second conduit (312) has a second outlet (302) to an ambient environment.

5. The respiratory support device (100) according to claim 3 or 4, wherein the first conduit (311) and the second conduit (312) are separated from each other.

6. The respiratory support device (100) according to claim 3 or 4, comprising
a valve (320) coupling the first conduit (311) and the second conduit (312) to each other,
wherein the valve (320) is configured to be in a first position (321) and in a second position (322),
wherein, in the first position (321), the valve (320) allows the first flow of air (211) through the first conduit (311) to the first outlet (201), and blocks the second flow of air (212) from flowing through the second conduit (312),
wherein, in the second position (322), the valve (320) allows the second flow of air (212) through the second conduit (312) and the water collection unit (208), and blocks the first flow of air (211) from flowing through the first outlet (201).

7. The respiratory support device (100) according to claim 6,
wherein the conduit system (200) comprises an inlet (214),
wherein the conduit system (200) is adapted to convey the first flow of air (211) from the inlet (214) via the valve (320) to the humidifier (204), and from the humidifier (204) to the first outlet (201),
wherein the conduit system (200) is adapted to convey the second flow of air (212) from the inlet (214) via the valve (320) to the water collection unit (208).

8. The respiratory support device (100) according to claim 7, comprising
a water conduit (216) connecting the water collection unit (208) and the humidifier (204) to each other,
water control unit (510); and
a water level sensor (502) configured to generate a water level signal (511) representative of a water level in the humidifier (204),
wherein the water conduit (216) is arranged to provide water from the water collection unit (208) to the humidifier (204).
wherein the water control unit (510) is configured to receive the water level signal (511),
wherein the water control unit (510) is configured to provide a valve control signal (512) (512) to the valve (320) in response to the water level signal (511) to control a position of the valve (320).

9. The respiratory support device (100) according to claim 8, wherein the water control unit (510) is configured to provide the valve control signal (512) to position the valve (320) in the first position (321) in case the water level signal (511) represents a water level above a threshold level,
wherein the water control unit (510) is configured to provide the valve control signal (512) to position the valve (320) in the second position (322) in case the water level signal (511) represents a water level below a threshold level.

10. The respiratory support device (100) according to claim 8 or 9, wherein the water control unit (510) is configured to:
receive a use signal (513) representative of the subject (110) is about to use or uses the respiratory support device (100),
in response to receiving the use signal (513), provide the valve control signal (512) to position the valve (320) in the first position (321).

11. The respiratory support device (100) according to any one of claims 1-10, wherein the water collection unit (208) comprises hygroscopic material (700) configured to adsorb water vapor from the second flow of air (212),
wherein respiratory support device (100) comprises a heater (506) arranged to heat the hygroscopic material (700) to either remove adsorbed water vapor from the hygroscopic material (700) or to adsorb water in the hygroscopic material (700), and wherein the humidifier (204) comprises a water reservoir (209) for storing water, wherein the heater (506) is arranged to provide heat to the water reservoir (209) to generate water vapor.

12. A method for using a respiratory support device (100) for providing air to a subject (110) via a patient interface (102), the method comprising:
generating (901) a first flow of air (211) via a first outlet (201) coupled to the patient interface (102);
using (902) a blower (206) to generate a second flow of air (212);
collecting (903) water from the second flow of air (212);
humidifying (904) the first flow of air (211) with the water.

13. The method according to claim 12, wherein the respiratory support device (100) comprising a humidifier (204) having a water reservoir (209);
wherein the method comprises:
using (905) the blower (206) to generate the second flow of air (212) before generating the first flow of air (211);
filling (906) the water reservoir (209) with water by collecting water from the second flow of air (212) till a water level in the water reservoir (209) reaches a threshold level;
after (907) the water level reaches the threshold level, stop using the blower (206) to generate the second flow of air (212), and start generating the first flow of air (211).

14. A computer program product, comprising instructions which, when executed by a control unit (210) of a respiratory support device (100) according to any one of claims 1-11, cause the respiratory support device (100) to perform the method of claim 12 or 13.

15. Water collection unit (208) for use in the respiratory support device (100) according to any one of claims 1-11, comprising a coupling device (704) adapted to couple with the conduit system (200).
